(19) Europäisches Patentamt
European Patent Office
Office européen des brevets

(11) Publication number: **0 498 988 A2**

# (12) EUROPEAN PATENT APPLICATION

(21) Application number: **91310311.5**

(22) Date of filing: **07.11.91**

(51) Int. Cl.[5]: **C08G 65/32**, C07C 45/00, B01J 25/02

(30) Priority: **11.02.91 US 653220**

(43) Date of publication of application: **19.08.92 Bulletin 92/34**

(84) Designated Contracting States: **DE FR GB**

(71) Applicant: **TEXACO CHEMICAL COMPANY**
**3040 Post Oak Boulevard**
**Houston, Texas 77056(US)**

(72) Inventor: **Sanderson, John Ronald**
**P O Box 587**
**Leander, TX 78641(US)**
Inventor: **Larkin, John Michael**
**12414 Dorsett Road**
**Austin, TX 78727(US)**

(74) Representative: **Brock, Peter William et al**
**UROUHART-DYKES & LORD 91 Wimpole Street**
**London W1M 8AH(GB)**

## (54) Catalytic process for the production of diketone derivatives of polyoxypropylene glycols.

(57) Polyoxypropylene glycols having molecular weights from 130 to 4000 can be converted into corresponding polyoxypropylene diketones, with good selectivity, using as catalyst Raney nickel or an unsupported nickel-copper-chromium catalyst containing, on an oxide-free basis 60 to 85 mole % of nickel, 14 to 37 mole % of copper, and 1 to 5 mole % of chromium.

EP 0 498 988 A2

This invention relates to an improved process for the production of diketones from polyoxypropylene glycols. More particularly, this invention relates to a catalytic process for the substantially selective conversion of polyoxypropylene glycols into diketones, wherein the polyoxypropylene glycol is brought into contact with catalyst selected from nickel, copper, chromium catalyst and Raney nickel, preferably at a temperature of 200° to 260°C and a pressure of 0.1 to 35 MPa (atmospheric to 5000 psig).

An article in the Journal of Organic Chemistry, 151, 5482 (1986) discloses that alkanones can be prepared by refluxing an alkanol in solution in benzene or toluene in the presence of a Raney nickel catalyst.

US-A-4960948 discloses the preparation of ketone terminated derivatives of polyoxypropylene glycols by oxidizing the polyoxypropylene glycol with a hypochlorite in solution in acetic acid.

Catalysts containing nickel, copper and chromium have been used for a variety of processes, including the preparation of N-alkyl substituted piperazines, or of morpholines, for reacting a glycol ether with an amine; for the conversion of hydroxy-containing feedstocks into the corresponding acyclic, cyclic or heterocyclic amines; and for reducing peroxide and hydroperoxide contaminants present in tert. butyl alcohol.

It has been discovered, in accordance with the present invention, that polyoxypropylene glycols can be converted into ketone terminated derivatives by the process described herein using a nickel, copper, chromia catalyst or Raney nickel catalyst.

The feed material for the present invention is a polyoxypropylene glycol, preferably having an average molecular weight of 130 to 4,000, and more preferably 200 to 2,000 and having formula (I):

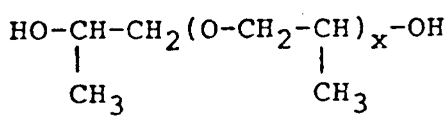

wherein x is 1 to 70.

Examples of such polyoxypropylene glycols include commercial products, such as a polyoxypropylene glycol having an average molecular weight of about 200, of about 500, of about 2000 or of about 4,000.

The reaction is suitably conducted under comparatively moderate conditions of temperature, e.g. of 200 to 260°C and at a pressure of 0.1 to 35 MPa (atmospheric to 5000 psig) total pressure.

The dehydrogenation catalyst to be used in accordance with the present invention is a Raney nickel or an unsupported nickel, copper, chromium catalyst. The preferred catalyst for use in this process is one containing nickel, copper and chromium. The catalyst is described in US-A-3152998 or -3654370. It is prepared by the reduction of a mixture of the oxides of nickel, copper and chromium in the presence of hydrogen at a temperature of 250 to 400°C. On an oxide-free basis, the catalyst contains 60 to 85 mole % of nickel, 14 to 37 mole % of copper and 1 to 5 mole % of chromium. A particularly preferred composition is one containing 70 to 80 mole % of nickel, 20 to 25 mole % of copper and 1 to 5 mole % of chromium, most preferably 72 to 78 mole % of nickel, 20 to 25 mole % of copper, and 1 to 3 mole % of chromium.

The process of the present invention may be conducted batchwise using an autoclave containing powdered catalyst, or it may be conducted continuously, by passing the feed materials over a bed of pelleted catalyst.

Normally, the desired diketone can be recovered from the reaction mixture without distillation. Fractional distillation may be necessary with lower molecular weight feedstocks.

The present invention is further illustrated by the following specific Examples.

Procedure

A series of continuous runs was carried out using a polyoxypropylene glycol having an average molecular weight of about 2000 (PPG-2000) as a feedstock. The reactor was a 75 x 1.3 cm stainless steel tube. The catalyst bed was 100 $cm^3$ and contained pellets of the catalyst to be evaluated. Liquid feed was pumped through the bottom of the reactor. Pressure regulation was with a Skinner Uni-flow valve and a Foxboro controller. Liquid was pumped with a Ruska pump. The reactor was electrically heated. 100 to 200 $cm^3$ of Prerun was collected at each temperature and then 100 to 200 $cm^3$ of material for analysis. The catalysts that were evaluated and the results are shown in the attached Tables. Runs 1 to 6 of Tables A and B illustrate the invention while Runs 7 to 13 are provided for comparison

TABLE A

| Run No. | Catalyst | Rate (cm3/h) | Pressure MPa | Temp (°C) | OH No. (mg/g) | Acid No. (mg/g) | Water (wt.%) |
|---|---|---|---|---|---|---|---|
| 1-1 | Ni/Cu/Cr | 100 | 0.8 | 220 | 38 | 0.63 | 0.16 |
| -2 | | | | 240 | 22.1 | 1.01 | 0.16 |
| -3 | | | | 260 | 15.4 | 0.24 | 0.216 |
| -4 | | | | 280 | 14.5 | 1.43 | 1.66 |
| 2-1 | Ni/Cu/Cr New charge | 100 | 0.8 | 200 | 44.6 | 0.78 | 0.167 |
| -2 | | | | 220 | 23.4 | 1.64 | 0.141 |
| -3 | | | | 240 | 26.6 | 1.18 | 0.183 |
| -4 | | | | 260 | 23.2 | 1.35 | 0.176 |
| 3-1 | Continued from above | 50 | 0.8 | 200 | 44.1 | 0.28 | 0.163 |
| -2 | | | | 220 | 22.4 | 0.60 | 0.257 |
| -3 | | | | 240 | 14.3 | 0.47 | 0.214 |
| -4 | | | | 260 | 8.03 | 0.93 | 0.165 |
| 4-1 | Continued from above | 100 | 3.55 | 200 | 65.3 | 0.64 | 0.196 |
| -2 | | | | 220 | 53.8 | 0.62 | 0.180 |
| -3 | | | | 240 | 30.9 | 1.11 | 0.213 |
| -4 | | | | 260 | 33.9 | 1.12 | 0.144 |
| 5-1 | Ni/Cu/Cr New charge | 100 | 0.8 | 220 | 34.0 | 1.84 | 2.42 |
| -2 | | | | 240 | 25.8 | 1.23 | 0.387 |
| -3 | | | | 260 | 7.16 | - | 0.293 |
| 6-1 | Ni/Cu/Cr Same catalyst Different batch | 100 | 0.8 | 220 | 36.7 | 1.16 | 0.871 |
| -2 | | | | 240 | 23.9 | 1.06 | 0.275 |
| -3 | | | | 260 | 17.8 | - | 0.131 |
| 7-1 | Ni/Cu/Cr/Fe kieselguhr | 100 | 0.8 | 220 | - | - | - |
| -2 | | | | 240 | 26.4 | 1.11 | 0.357 |
| -3 | | | | 260 | 36.9 | 1.29 | 0.141 |
| -4 | | | | 280 | 26.5 | 2.19 | 0.082 |

**TABLE B**

| N.B. No. | Catalyst | Rate (cm3/h) | Pressure MPa | Temp (°C) | OH No. (mg/g) | Acid No. (mg/g) | Water (wt.%) |
|---|---|---|---|---|---|---|---|
| 8-1 | Ni/Cu/Cr on $Al_2O_3$ | 100 | 0.8 | 220 | 52.82 | 0.095 | 0.597 |
| -2 | | | | 240 | 45.30 | 0.88 | 0.465 |
| -3 | | | | 260 | 55.3 | 1.05 | 0.783 |
| -4 | | | | 280 | 56.5 | 1.46 | 1.274 |
| 9-1 | Ni/Cu/Cr on $Al_2O_3$ Mo promoted | 100 | 0.8 | 160 | 87 | 0.28 | 0.39 |
| -2 | | | | 180 | 56 | 0.80 | 0.20 |
| -3 | | | | 200 | 48 | 0.62 | 0.55 |
| -4 | | | | 220 | 32 | 0.55 | 0.62 |
| 10-1 | Ni/Cu/Cr on $Al_2O_3$ Mo promoted | 100 | 0.8 | 220 | 29.2 | 1.60 | 1.87(?) |
| -2 | | | | 240 | 43.0 | 1.20 | 0.40 |
| -3 | | | | 260 | 46.0 | 2.62 | 0.19 |
| -4 | | | | 280 | 36.0 | 2.39 | 0.16 |
| 11-1 | 0.5% Ru on C | 100 | 0.8 | 220 | 85.2 | 0.77 | 0.432 |
| -2 | | | | 240 | 84.4 | 0.50 | 0.432 |
| -3 | | | | 260 | 88.6 | 0.30 | 0.488 |
| -4 | | | | 280 | 101.0 | 0.74 | 0.584 |
| 12-1 | Copper Chromite 80% CuO, 20% $Cr_2O_3$, 1.5mm pellets | 100 | 0.8 | 220 | 49.6 | 3.54 | 0.582 |
| -2 | | | | 240 | 48.1 | 4.88 | 0.686 |
| -3 | | | | 260 | 41.1 | 16.3 | 1.896 |
| -4 | | | | 280 | 44.6 | 9.28 | 0.076 |
| 13-1 | Copper Chromite 46% CuO, 46% $Cr_2O_3$, 4% $MnO_2$ | 100 | 0.8 | 200 | 62.3 | 0.83 | 0.830 |
| -2 | | | | 220 | 56.3 | 0.64 | 0.221 |
| -3 | | | | 240 | 50.2 | 0.42 | 0.201 |
| -4 | | | | 260 | 44.9 | 1.49 | 0.376 |
| -5 | | | | 280 | 40.7 | 2.80 | 0.693 |
| -6 | | | | 300 | 25.1 | 1.25 | 0.504 |

## Discussion of Table A and Table B

Run 1 was the first conducted using the Ni/Cu/Cr catalyst. The optimum temperature appeared to be 260 ° C. Above 260 ° C, there was extensive decomposition as evidenced by the jump in acid number and the jump in the percentage water present.

The reactor was recharged with the same catalyst for Run 2 and the reaction was conducted at 200-260 ° C. The optimum temperature here appeared to be 240 to 260 ° C. The reaction was continued in Run 3

(using the same catalyst) and runs were again conducted at 200 to 260° C. Again, the optimum temperature is 260° C. The reaction was continued over several days and again examined at 200 to 260° C. In Run 4, the optimum temperature is again 240 to 260° C., but the catalyst appears to lose activity after long continuous use.

Run 5 was carried out using a new charge of the same catalyst, and excellent results are obtained at 260° C.

Run 6 was also carried out using a new charge of the same type of catalyst, but with slightly lower activity. The reason for the slight difference in results is probably due to differences in surface area.

Run 7 was with a catalyst comprising Ni/Cu/Cr/Fe on kieselguhr, and it gave only fair results.

Table B continues other catalysts for comparison with the Ni/Cu/Cr catalyst, employed according to this invention, and these include:

Ni/Cu/Cr on $Al_2O_3$,
Ni/Cu/Cr on $Al_2O_3$ (Mo promoted),
0.5% Ru on C, and
Copper chromite

None of the catalysts gives results which are as good as the results obtained with unsupported Ni/Cu/Cr catalyst. It is especially surprising that the copper chromite catalyst gives such poor results, since it is known in the art that copper chromite catalysts are good dehydrogenation catalysts.

TABLE C

| N.B. No. | Catalyst | Rate $cm^3/h$ | Pressure MPa | Temp (°C) | OH No. (mg/g) | Acid No. (mg/g) | Water (wt.%) |
|---|---|---|---|---|---|---|---|
| 14-1 | Copper Chromite, 1.5mm tablets, 42% CuO, 44% $Cr_2O_3$, 9% BaO | 100 | 0.8 | 220 | 55.92 | 1.35 | 0.073 |
| -2 | | | | 240 | 48.20 | 1.08 | 0.148 |
| -3 | | | | 260 | 41.1 | 0.86 | 0.363 |
| -4 | | | | 280 | 40.2 | 2.46 | 0.718 |
| 15-1 | Copper Chromite, 1.5mm tablets, 27% CuO, 32% $Cr_2O_3$, 6% BaO | 100 | 0.8 | 220 | 52.49 | 0.99 | 0.0296 |
| -2 | | | | 240 | 47.25 | 0.92 | 0.179 |
| -3 | | | | 260 | 54.88 | 1.09 | 0.341 |
| 16-1 | 0.5% Rh on $Al_2O_3$, 1.5mm pellets | 100 | 0.8 | 220 | 65.0 | 0.11 | 0.283 |
| -2 | | | | 240 | 67.0 | 0.48 | 0.331 |
| -3 | | | | 260 | 61.0 | 1.39 | 0.488 |
| -4 | | | | 280 | 75.2 | 2.70 | 1.382 |

For comparison.

Comparison catalysts continue in Table C. None of the copper chromite catalysts in Tablc C was as good as the Ni/Cu/Cr catalyst of this invention, and in fact, most are very poor catalysts for dehydrogenation of PPG. 0.5% Rh on alumina is also a very poor catalyst. All of these catalysts give $<25\%$ conversion under optimum conditions.

Raney Nickel

Table D shows runs with three different grades of Raney nickel. Under optimum conditions, conversions as high as 75% could be obtained. There is very little dehydration to olefin or oxidation to acid, as evidenced by the low percent water and low acid number.

TABLE D

| N.B. No. | Catalyst | Rate ($cm^3$/h) | Pressure (MPa) | Temp (°C) | OH No. (mg/g) | Acid No. (mg/g) | Water (wt.%) |
|---|---|---|---|---|---|---|---|
| 17-1 | Raney Granular Nickel | 100 | 100 | 220 | 31.3 | 0.92 | 0.616 |
| -2 | Surface | | | 240 | 25.5 | 0.98 | 0.189 |
| -3 | area = 31 $m^2$/g | | | 260 | 15.7 | 0.30 | 0.259 |
| -4 | | | | 280 | 25.7 | 1.09 | 0.466 |
| 18-1 | Raney Granular Nickel | 100 | 100 | 200 | 33.6 | 0.59 | 0.78 |
| -2 | Surface | | | 220 | 16.6 | 0.76 | 0.26 |
| -3 | area = 19 $m^2$/g | | | 240 | 14.1 | 0.94 | 0.11 |
| -4 | | | | 260 | 16.9 | 0.77 | 0.19 |
| 19-1 | Raney Granular Nickel | 100 | 100 | 200 | 23.5 | - | 0.40 |
| -2 | Surface | | | 220 | 17.7 | 0.33 | 0.15 |
| -3 | area = 22 m2/g | | | 240 | 22.5 | 0.32 | 0.17 |
| -4 | | | | 260 | 25.4 | 0.43 | 0.29 |

All these catalysts were 8x12 mesh (1.68x2.38mm)

It is to be observed that the hydroxyl number of the PPG-2000 starting material was 57.0. Cracking of the PPG-2000 is indicated when the reaction product has a hydroxyl number of more than 57. The extent to which the PPG-2000 was converted into the desired diketone product is indicated by the extent to which the hydroxyl number of the reaction product was lowered (the lower the hydroxyl number the greater the conversion).

The PPG starting material had an acid number of about zero. An increase in the acid number of the reaction product is a measure of the extent to which the terminal hydroxyl groups of the PPG-2000 were converted into undesired carboxyl acid groups rather than the desired ketone groups.

Dehydration of the PPG-2000 leads to the formation of undesired by-products. Therefore, the weight percentage of water in the reaction product is a measure of the extent to which undesired dehydration of the PPG-2000 occurs.

Turning now to the Tables, it is to be noted that the catalyst of the present invention was used in Runs 1 to 6.

Significant conversion of the PPG-2000 into the diketone derivatives was obtained, the conversion increasing with increases in the reaction temperature. The reaction products had comparatively low acid numbers and water contents. Good results were obtained with a Raney nickel catalys. Mixed results were obtained with the other catalysts.

Note that satisfactory conversions (as measured by hydroxyl number) were obtained with the catalysts of the present invention in runs 1-1, 2-2, 5-1 6-1, but that the use of an alumina support for the catalyst resulted in the formation of a reaction product characterized by low conversion to the diketone derivative.

When a modified supported nickel, copper, chromia, molybdenum catalyst was used, satisfactory results were obtained at a reaction temperature of 220° C (9-4). Note however, that unsatisfactory results were obtained at lower temperatures (9-1 and 9-2)

In run 10-1, the use of a phosphorus-promoted alumina support for the catalyst resulted in excessive dehydration at 220° C. In contrast to the results shown in Table A, an increase in reaction temperature resulted in increased undesirable production of carboxyl acid derivatives of decreased production of the desired diketone derivatives (see Table B).

The use of a ruthenium on activated carbon catalyst resulted in undesirable cracking (11-1).

The use of a chromia supported copper oxide catalyst in run 12-1 resulted in an undesirable conversion of the PP-2000 to carboxylic acid derivatives, whereas there was only a minor conversion of the PPG-2000 to the desired diketone derivatives in runs 13-2, 14-1, and 15-1.

In run 16-1, the use of an alumina supported rhenium catalyst resulted in cracking of the PPG-2000 feedstock.

## Claims

1. A process for the conversion of a polyoxypropylene glycol having an average molecular weight of 130 to 4,000 into the corresponding polyoxypropylene diketone by dehydrogenation in the presence of a catalyst characterized in that the catalyst comprises Raney nickel or an unsupported nickel- copper-chromium catalyst containing, on an oxide-free basis, 60 to 85 mole percent of nickel, 14 to 37 mole percent of copper and 1 to 5 mole percent of chromium.

2. A method according to Claim 1 characterized in that the catalyst comprises 72 to 78 mole percent of nickel, 20 to 25 mole percent of copper and 1 to 3 mole percent of chromium.

3. A method according to Claim 1 or 2 characterized in that the dehydrogenation is conducted at a temperature of 200 to 260° C and a pressure of 0.1 to 35 MPa (atmospheric to 5000 psig).

4. A process according to any one of Claims 1 to 3 characterized in that the polyoxypropylene glycol has an average molecular weight of 200 to 2,000.